# EUROPEAN PATENT APPLICATION

(11) **EP 2 481 738 A1**
(43) Date of publication of application: **01.08.2012**
(21) Application number: 10818572.9
(22) Date of filing: 27.09.2010
(51) Int. Cl.: C07D 413/14, A61K 31/536, A61P 1/02, A61P 1/04, A61P 1/16, A61P 1/18, A61P 7/00, A61P 7/02, A61P 9/10, A61P 11/00, A61P 11/06, A61P 13/12, A61P 15/06, A61P 17/00, A61P 17/02, A61P 17/06, A61P 19/02, A61P 25/00, A61P 27/02, A61P 29/00, A61P 31/04

(54) **MALEIC ACID SALT AND CRYSTAL THEREOF**

(30) Priority: 25.09.2009 JP 2009220296
(71) Applicant: Kyorin Pharmaceutical Co., Ltd., Tokyo 101-8311 (JP)
(72) Inventor: TSUBUKI, Takeshi, Shimotsuga-gun Tochigi 329-0114 (JP); SAGAWA, Tetsuya, Shimotsuga-gun Tochigi 329-0114 (JP); FUNADA, Keiko, Shimotsuga-gun Tochigi 329-0114 (JP); ARAYA, Ichiro, Shimotsuga-gun Tochigi 329-0114 (JP)
(74) Representative: Vossius & Partner
(86) International application number: PCT/JP2010/005786
(87) International publication number: WO 2011/036895

(57) **Abstract**

Disclosed is a benz[d][1,3]oxazine derivative having excellent stability when used as an active ingredient for a medicinal agent, and also having excellent solubility and crystallinity. Specifically disclosed is 2-[2-((S)-3-dimethylaminopyrrolidin-1-yl)pyridin-3-yl]-5-ethyl-7-methoxy-4H-benz [d][1,3]oxazin-4-one maleate.

## Description

### Technical Field

The present invention relates to a maleic acid salt of a benz[d][1,3]oxazine derivative, and particularly, to a crystal thereof.

### Background Art

Human neutrophil elastase is a kind of serine hydrolase released from granules of neutrophil, which appear during infection or inflammatory disease. Neutrophil elastase is an enzyme hydrolyzing elastin, collagen, proteoglycan, fibronectin, and other proteins which constitute the interstitium of intravital connective tissues such as lung, cartilage, vascular wall, and skin. In addition, it has been clarified that neutrophil elastase acts on other proteins or cells.

Serine hydrolase including neutrophil elastase maintains homeostasis in the living body. The activity of serine hydrolase is regulated by an endogenous protein inhibitor such as an α1-protease inhibitor, an α2-macroglobulin, or a secretory leukocyte protease inhibitor. However, when a balance between the neutrophil elastase and the endogenous inhibitor is lost by excessive release of neutrophil elastase in the inflammation site or by decline in an inhibitor level, the regulation of neutrophil elastase activity cannot be kept, and tissues are injured.

Examples of diseases involved in serine hydrolase include pulmonary emphysema, acute respiratory distress syndrome, adult respiratory distress syndrome (ARDS), idiopathic interstitial pneumonia (IIP), cystic pulmonary fibrosis, chronic interstitial pneumonia, chronic bronchitis, chronic sinopulmonary infection, diffuse panbronchiolitis, bronchiectasis, asthma, pancreatitis, nephritis, hepatic failure, chronic rheumatoid arthritis, joint scleroma, osteoarthritis, psoriasis, periodontitis, atherosclerosis, rejection against organ transplant, premature amniorrhexis, bullous dermatosis, shock, sepsis, systemic lupus erythematosus (SLE), Crohn's disease, disseminated intracapillary coagulation (DIC), tissue injury after ischemia-reperfusion, formation of cornea cicatricial tissue, myelitis, and the like. As a therapeutic agent for these diseases, the development of serine hydrolase inhibitor is expected.

As an excellent serine hydrolase inhibitor, a benz[d][1,3]oxazine derivative has been reported (Patent Literature 1).

### Citation List

### Patent Literature

Patent Literature 1: WO2008/036379

### Summary of Invention

### Technical Problem

The benz[d][1,3]oxazine derivative described in Patent Literature 1 is excellent in serine hydrolase inhibitory activity but has low long-term storage stability, and therefore a compound having more excellent physical properties is expected from the viewpoint of manufacturing pharmaceuticals as a drug substance for manufacture. An object of the present invention is to provide a benz[d][1,3]oxazine derivative having excellent stability as a drug substance, solubility, and crystallinity.

### Solution to Problem

The present inventors have intensively studied to achieve the object, and found that a maleic acid salt of a benz[d][1,3]oxazine derivative has excellent stability as a drug substance, solubility, and crystallinity, thereby completing the present invention.
The summary of the present invention is as follows:

1)

Maleic acid salt of 2-[2-((S)-3-dimethylaminopyrrolidin-1-yl)pyridin-3-yl]-5-ethyl-7-methoxy-4H-benz [d][1,3]oxazin-4-one represented by the formula (1),

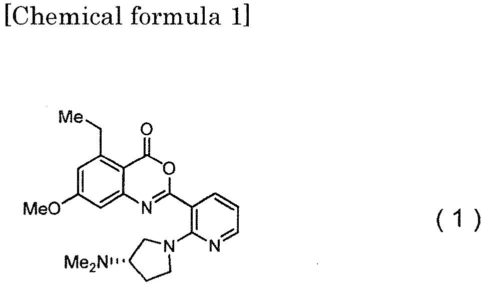

2) A crystal of 2-[2-((S)-3-dimethylaminopyrrolidin-1-yl)pyridin-3-yl]-5-ethyl-7-methoxy-4H-benz [d][1,3]oxazin-4-one maleate.

3) A crystal of 2-[2-((S)-3-dimethylaminopyrrolidin-1-yl)pyridin-3-yl]-5-ethyl-7-methoxy-4H-benz [d][1,3]oxazin-4-one monomaleate.

4) The crystal of 2-[2-((S)-3-dimethylaminopyrrolidin-1-yl)pyridin-3-yl]--5-ethyl-7-methoxy-4H-benz [d][1,3]oxazin-4-one monomaleate according to 3), having a characteristic peak at a diffraction angle (2θ ± 0.5°) of 10.8° in powder x-ray diffraction.

5) The crystal of 2-[2-((S)-3-dimethylaminopyrrolidin-1-yl)pyridin-3-yl]-5-ethyl-7-methoxy-4H-benz [d][1,3]oxazin-4-one monomaleate according to 3), having a characteristic peak at a diffraction angle (2θ ± 0.5°) of 14.8° in powder x-ray diffraction.

6) The crystal of 2-[2-((S)-3-dimethylaminopyrrolidin-1-yl)pyridin-3-yl]-5-ethyl-7-methoxy-4H-benz [d][1,3]oxazin-4-one monomaleate according to 4), wherein melting point thereof measured by thermal analysis TG-DTA is about 153°C.

7) The crystal of 2-[2-((S)-3-dimethylaminopyrrolidin-1-yl)pyridin-3-yl]-5-ethyl-7-methoxy-4H-benz [d][1,3]oxazin-4-one monomaleate according to 5), wherein melting point thereof measured by the thermal analysis TG-DTA is about 167°C.

8) A mixture of a crystal of 2-[2-((S)-3-dimethylaminopyrrolidin-1-yl)pyridin-3-yl]-5-ethyl-7-methoxy-4H-benz [d][1,3]oxazin-4-one monomaleate having a characteristic peak at a diffraction angle (2θ ± 0.5°) of 10.8° in powder x-ray diffraction, and
   a crystal of 2-[2-((S)-3-dimethylaminopyrrolidin-1-yl)pyridin-3-yl]-5-ethyl-7-methoxy-4H-benz [d][1,3]oxazin-4-one monomaleate having a characteristic peak at a diffraction angle (2θ ± 0.5°) of 14.8° in powder x-ray diffraction.

9) A method for producing the crystal of 2-[2-((S)-3-dimethylaminopyrrolidin-1-yl)pyridin-3-yl]-5-ethyl-7-methoxy-4H-benz [d][1,3]oxazin-4-one monomaleate according to 4), comprising the following steps (i) and (ii):
   (i) a step of dissolving a 2-[2-((S)-3-dimethylaminopyrrolidin-1-yl)pyridin-3-yl]5-ethyl-7-methoxy-4H-benz [d][1,3]oxazin-4-one free base in acetone to produce a solution; and
   (ii) a step of adding a solution of maleic acid in acetone to the solution obtained in the step (i).

10) A method for producing the crystal of 2-[2-((S)-3-dimethylaminopyrrolidin-1-yl)pyridin-3-yl]-5-ethyl-7-methoxy-4H-benz [d][1,3]oxazin-4-one monomaleate according to 4), comprising
   using an aprotic organic solvent as a solvent in which a 2-[2-((S)-3-dimethylaminopyrrolidin-1-yl)pyridin-3-yl]-5-ethyl-7-methoxy-4H-benz [d][1,3]oxazin-4-one free base and maleic acid are dissolved, and
   using one or more diluting solvents selected from the group consisting of ethyl acetate, t-butyl methyl ether, tetrahydrofuran, diethyl ether, and diisopropyl ether as a solvent to be added for precipitation of crystals.

11) A method for recrystallizing 2- [2-((S)-3-dimethylaminopyrrolidin-1-yl)pyridin-3-yl]-5-ethyl-7-methoxy-4H-benz [d][1,3]oxazin-4-one monomaleate, comprising the following steps (i) to (iii):
   (i) a step of dissolving a 2- [2-((S)-3-dimethylaminopyrrolidin-1-yl)pyridin-3-yl]-5-ethyl-7-methoxy-4H-benz [d][1,3]oxazin-4-one monomaleate in acetone and water to produce a solution;
   (ii) a step of adding t-butyl methyl ether or diisopropyl ether to the solution obtained in the step (i); and
   (iii) a step of isolating crystalline solid crystallized by the step (ii).

12) A pharmaceutical containing the crystal of 2- [2-((S)-3-dimethylaminopyrrolidin-1-yl)pyridin-3-yl]-5-ethyl-7-methoxy-4H-benz [d] [1,3]oxazin-4-one monomaleate according to 3) as a drug substance. Advantageous Effects of Invention

The present invention can provide a drug substance having excellent stability, solubility, and crystallinity. In particular, it is useful for manufacture of injections because the maleic acid salt of the present invention has water solubility as high as 10 mg/mL or higher.

### Brief Description of Drawings

[Fig. 1] a thermal analysis TG-DTA chart of 2-[2-((S)-3-dimethylaminopyrrolidin-1-yl)pyridin-3-yl]-5-ethyl-7-methoxy-4H-benz [d][1,3]oxazin-4-one monomaleate (low melting point crystal).
[Fig. 2] a powder x-ray diffraction pattern of 2-[2-((S)-3-dimethylaminopyrrolidin-1-yl)pyridin-3-yl]-5-ethyl-7-methoxy-4H-benz [d][1,3]oxazin-4-one monomaleate (low melting point crystal). The longitudinal axis indicates an intensity (cps), and the horizontal axis indicates a diffraction angle (2θ)[°].
[Fig. 3] a thermal analysis TG-DTA chart of 2-[2-((S)-3-dimethylaminopyrrolidin-1-yl)pyridin-3-yl]-5-ethyl-7-methoxy-4H-benz [d][1,3]oxazin-4-one monomaleate (high melting point crystal).
[Fig. 4] a powder x-ray diffraction pattern of 2-[2-((S)-3-dimethylaminopyrrolidin-1-yl)pyridin-3-yl]-5-ethyl-7-methoxy-4H-benz [d][1,3]oxazin-4-one monomaleate (high melting point crystal). The longitudinal axis indicates an intensity (cps), and the horizontal axis indicates a diffraction angle (2θ)[°].
[Fig. 5] a thermal analysis TG-DTA chart of 2-[2-((S)-3-dimethylaminopyrrolidin-1-yl)pyridin-3-yl]-5-ethyl-7-methoxy-4H-benz [d][1,3]oxazin-4-one hydrochloride monohydrate Lot 1.
[Fig. 6] a thermal analysis TG-DTA chart of 2-[2-((S)-3-dimethylaminopyrrolidin-1-yl)pyridin-3-yl]-5-ethyl-7-methoxy-4H-benz [d][1,3]oxazin-4-one hydrochloride monohydrate Lot 2.
[Fig. 7] a thermal analysis TG-DTA chart of 2-[2-((S)-3-dimethylaminopyrrolidin-1-yl)pyridin-3-yl]-5-ethyl-7-methoxy-4H-benz [d][1,3]oxazin-4-one hydrochloride monohydrate Lot 3.
[Fig. 8] a powder x-ray diffraction pattern of 2-[2-((S)3-dimethylaminopyrrolidin-1-yl)pyridin-3-yl]-5-ethyl-7-methoxy-4H-benz [d][1,3]oxazin-4-one hydrochloride monohydrate. The longitudinal axis indicates an intensity (cps), and the horizontal axis indicates a diffraction angle (2θ)[°]. Description of Embodiments

Maleic acid salt of 2- [2-((S)-3-dimethylaminopyrrolidin-1-yl)pyridin-3-yl]-5-ethyl-7-methoxy-4H-benz [d][1,3]oxazin-4-one in the specification is not particularly limited as long as it is stable enough to be used as a drug substance for manufacturing pharmaceuticals, and includes a crystal and a crystal containing a solvent. Examples of 2-[2-((S)-3-dimethylaminopyrrolidin-1-yl)pyridin-3-yl]-5-ethyl-7-methoxy-4H-benz [d][1,3]oxazin-4-one monomaleate (hereinafter simply referred to as monomaleic acid salt) include a low melting point crystal, a high melting point crystal, and a mixture of low and high melting point crystals as described below. Since the maleic acid salt of the present invention has excellent solubility, stability, and crystallinity, it is useful as a drug substance for pharmaceuticals. Further, since the monomaleic acid salt of the present invention does not have adhesion water, the weight thereof is not increased or decreased by moisture absorption or desorption. Accordingly, it is excellent in handleability.

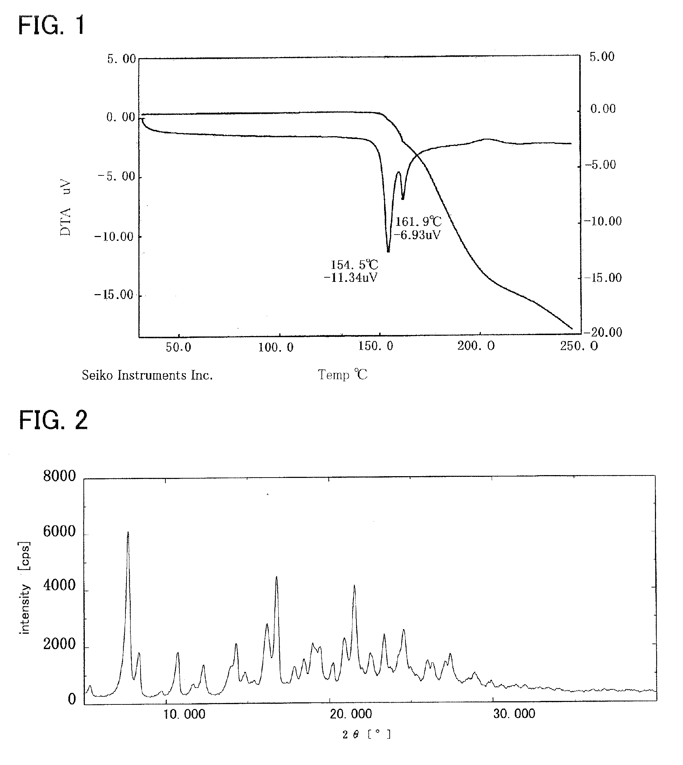

There exists a crystal that shows a powder x-ray diffraction pattern having peaks at diffraction angles (2θ ± 0.5°) of 7.80°, 10.8°, 16.2°, 16.9°, and 21.6° as shown in Fig. 2, when the crystal of 2-[2-((S)-3-dimethylaminopyrrolidin-1-yl)pyridin-3-yl]-5-ethyl-7-methoxy-4H-benz [d][1,3]oxazin-4-one monomaleate is measured using copper radiation. In particular, the crystal has a characteristic powder x-ray diffraction peak, which can be distinguished from other crystals, at a diffraction angle (2θ ± 0.5°) of 10.8°.
Further, from the measurement by the thermal analysis TG-DTA (Thermogravimetry-Differential Thermal Analysis), the melting point of the crystal is about 153°C. Furthermore, the melting point of the crystal determined by the thermal analysis TG-DTA is lower than that of a high melting point crystal described below. For this reason, in the specification, the crystal is referred to as a low melting point crystal of 2-[2-((S)-3-dimethylaminopyrrolidin-1-yl)pyridin-3-yl]-5-ethyl-7-methoxy-4H-benz [d][1,3]oxazin-4-one monomaleate (Also, simply referred to as low melting point crystal).

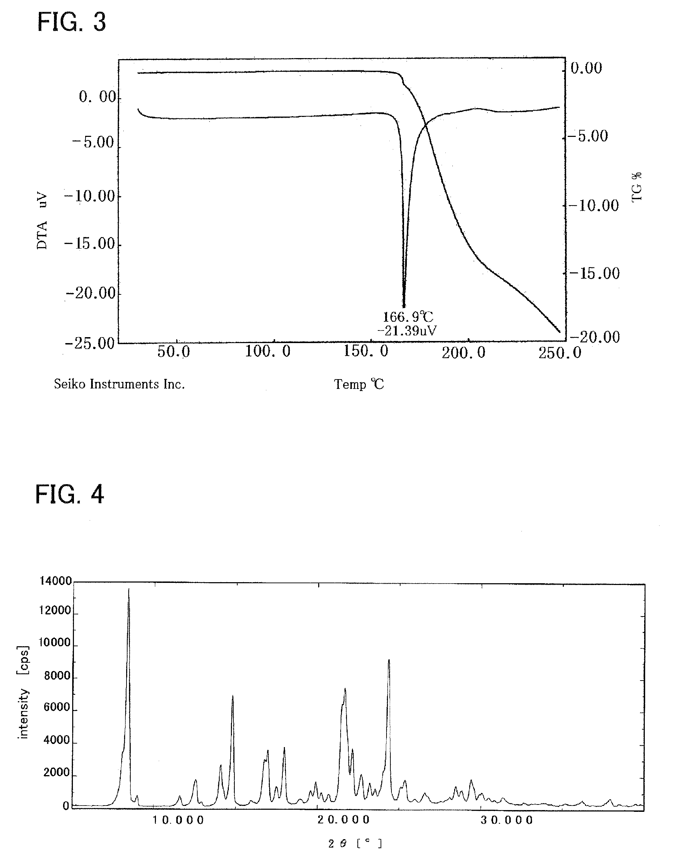

In addition, there exists a crystal that shows a powder x-ray diffraction pattern having peaks at diffraction angles (2θ ± 0.5°) of 8.38°, 14.8°, 21.5°, 21.7°, and 24.3° as shown in Fig. 4, when the crystal of 2-[2-((S)-3-dimethylaminopyrrolidin-1-yl)pyridin-3-yl]-5-ethyl-7-methoxy-4H-benz [d][1,3]oxazin-4-one monomaleate is measured using copper radiation. In particular, the crystal has a characteristic powder x-ray diffraction peak which can be distinguished from other crystals at a diffraction angle (2θ ± 0.5°) of 14.8°.
Further, from the measurement by the thermal analysis TG-DTA, the melting point of the crystal is about 167°C. Furthermore, the melting point of the crystal determined by the thermal analysis TG-DTA is higher than that of the low melting point crystal described above. Thus, the crystal is referred to as a high melting point crystal of 2- [2-((S)-3-dimethylaminopyrrolidin-1-yl)pyridin-3-yl]-5-ethyl-7-methoxy-4H-benz [d][1,3]oxazin-4-one monomaleate (Also,simply referred to as high melting point crystal).

A low melting point crystal is transformed into a high melting point crystal under warming through phase transition. The high melting point crystal has excellent crystallinity and stability as compared with the low melting point crystal.

The present invention may be a mixture of the low melting point crystal and the high melting point crystal. From the measurement by the thermal analysis TG-DTA, the melting point of the mixture on a low melting point crystal side is 152 to 157°C. From the measurement by the thermal analysis TG-DTA, the melting point of the mixture on a high melting point crystal side is 160 to 167°C.

Maleic acid salt of 2-[2-((S)-3-dimethylaminopyrrolidin-1-yl)pyridin-3-yl]-5-ethyl-7-methoxy-4H-benz [d][1,3]oxazin-4-one can be produced using a solvent in which maleic acid and 2-[2-((S)-3-dimethylaminopyrrolidin-1-yl)pyridin-3-yl]-5-ethyl-7-methoxy-4H-benz [d][1,3]oxazin-4-one are dissolved.
The solvent used in the production is preferably an aprotic organic solvent such as acetonitrile, ethyl acetate, acetone, or tetrahydrofuran. In particular, the solvent is more preferably acetone. A diluting solvent may be further added to a suspension of crystallized crystalline solid. The diluting solvent is preferably ethyl acetate, t-butyl methyl ether, tetrahydrofuran, diethyl ether, or diisopropyl ether. In particular, the diluting solvent is more preferably ethyl acetate, t-butyl methyl ether, or diisopropyl ether.

Furthermore, it is preferable that 2-[2-((S)-3-dimethylaminopyrrolidin-1-yl)pyridin-3-yl]-5-ethyl-7-methoxy-4H-benz [d][1,3]oxazin-4-one maleate be recrystallized using a suitable solvent. As a solvent for recrystallization, acetone, tetrahydrofuran, acetonitrile, 1,2-dimethoxyethane, ethyl acetate, or water can be used alone or in combination. Among them, an acetone-water mixed solvent is preferable. Examples of a diluting solvent to be added for precipitation of crystals include isopropyl ether, ethyl acetate, and t-butyl methyl ether.

### [Examples]

The present invention will be described in detail by Examples, and the present invention is not limited to these Examples.
Thermal analysis TG-DTA and powder x-ray diffractrion were performed under the following conditions.

Thermal analysis TG-DTA:
A sample (5 mg) was placed in a sample pan (a container made of aluminum), and thermal analysis TG-DTA of the sample was performed using EXSTAR 6000 thermal analyzer manufactured by Seiko Instruments Inc.

### Measurement Conditions

Rate of temperature increase: 5°C/min
Atmosphere: nitrogen Flow rate: 100 mL/min

Powder x-ray diffractrion:
A sample (about 100 mg) was (lightly) pulverized in an agate mortar, and a packed section of a sample flat holder made of glass was filled with the pulverized sample. Powder x-ray diffraction of the sample was then performed using RINT2200 powder x-ray diffractometer (Rigaku Corporation).

### Measurement Conditions

Tube current: 36 mA
Tube voltage: 40 kV
Scanning rate: 2°/min
Wavelength: 1.5405 Å (Kα1), 1.5443 Å (Kα2), 1.3922 Å (Kβ1)
Divergence slit: 1°
Receiving slit: 0.15 mm
Scattering slit: 1°
Anticathode: copper
Scanning range: 5 to 40°

### [Example 1]

Synthesis of low melting point crystal using acetone and ethyl acetate as solvent for crystallization
A solution of maleic acid (1.87 g) in acetone (15 mL) was mixed in a solution of 2-[2-((S)-3-dimethylaminopyrrolidin-1-yl)pyridin-3-yl]-5-ethyl-7-methoxy-4H-benz [d][1,3]oxazin-4-one (6.05 g) in acetone (60.5 mL). (Hereinafter, the prepared solution was referred to as a mixed solution. The same is applied to Example 2.) A container (an inflow line) was washed with 15 mL of acetone, and then the washing solution was added to the mixed solution. The mixed solution was stirred at room temperature to crystallize a reaction product (monomaleic acid salt). After that, 181 mL of ethyl acetate was added to the mixed solution, and the mixture was stirred under heating at an internal temperature of 50 to 53°C for 1 hour. The mixed solution was stirred under cooling at room temperature, and then stirred at an internal temperature of 22 to 30°C for 1 hour. Subsequently, the precipitated solid was collected by filtration, and was washed with 30 mL of ethyl acetate to obtain 11.2 g of wet crystal.

The crystal was dried with blowing air at 50°C to obtain 6.35 g (yield: 81%) of yellowish white cakes and powder of 2-[2-((S)-3-dimethylaminopyrrolidin-1-yl)pyridin-3-yl]-5-ethyl-7-methoxy-4H-benz [d][1,3]oxazin-4-one monomaleate.

### Melting point (hot plate method): 153 to 155°C (melting)

As seen from the results of thermal analysis TG-DTA (Fig. 1) and powder x-ray diffractrion (Fig. 2), the monomaleic acid salt in Example 1 was a low melting point crystal.

### [Example 2]

Synthesis of low melting point crystal using acetone and t-butyl methyl ether as solvent for crystallization
A solution of 2-[2-((S)-3-dimethylaminopyrrolidin-1-yl)pyridin-3-yl]-5-ethyl-7-methoxy-4H-benz [d][1,3]oxazin-4-one (10.0 g) in acetone (180 mL) was stirred at an external temperature of 30°C. 3.09 g of maleic acid was added at an internal temperature of 30°C, and a container (an inflow line) was washed with 20 mL of acetone, and then the washing solution was added to the mixed solution. The mixture was stirred to crystallize a reaction product (monomaleic acid salt). The mixed solution was stirred for 10 minutes, 200 mL of t-butyl methyl ether was added to the mixed solution, and the mixture was stirred under heating at an internal temperature of 45 to 50°C for 1 hour. The mixed solution was stirred under cooling, and further stirred at an internal temperature of 15°C or lower (to an internal temperature of 6°C) for 10 minutes. The precipitated crystal was collected by filtration, and washed with 50 mL of t-butyl methyl ether.

A wet crystal was dried with blowing air at 50°C to obtain 11.7 g (yield: 91%) of white flocculent crystal of 2-[2-((S)-3-dimethylaminopyrrolidin-1-yl)pyridin-3-yl]-5-ethyl-7-methoxy-4H-benz [d][1,3]oxazin-4-one monomaleate.
Melting point (hot plate method): 154 to 155°C (melting)

### [Example 3]

Phase transition of low melting point crystal to high melting point crystal 2.04 g of 2-[2-((S)-3-dimethylaminopyrrolidin-1-yl)pyridin-3-yl]-5-ethyl-7-methoxy-4H-benz [d][1,3]oxazin-4-one monomaleate which was a low melting point crystal obtained in Example 1 was lightly pulverized with an agate mortar, and stood in a blow dryer at 130°C. Subsequently, the maleic acid salt stood in the blow dryer for 4 hours to prepare a specimen while the maleic acid salt was stirred every half hour.
Melting point before heating (hot plate method): 153 to 154°C (melting)
Melting point after 4-hour heating (hot plate method): 165 to 166°C (decomposition)
As seen from the results of thermal analysis TG-DTA (Fig. 3) and powder x-ray diffractrion (Fig. 4), the specimen was a high melting point crystal.

### [Example 4]

Recrystallization using acetone, water, and t-butyl methyl ether (low melting point crystal + high melting point crystal)
200 mL of acetone and 10 mL of water were added to 10.0 g of 2-[2-((S)-3-dimethylaminopyrrolidin-1-yl)pyridin-3-yl]-5-ethyl-7-methoxy-4H-benz [d][1,3]oxazin-4-one monomaleate which was obtained in Example 2, and the mixture was dissolved under stirring at an external temperature of 45°C. This solution was subjected to hot filtration, and a filter was washed with a mixed solvent of 30 mL of acetone and 1.5 mL of water. While a filtrate (hereinafter also referred to as mixed solution) was stirred at an external temperature of 45°C, 50 mL of t-butyl methyl ether was added to the filtrate. The mixture was stirred under cooling to crystallize a monomaleic acid salt (crystallization starting temperature: internal temperature 27°C). The mixed solution containing the precipitated monomaleic acid salt was stirred at an internal temperature of 23 to 27°C for 30 minutes, and then stirred under heating. Thus, most of precipitated crystal was dissolved in the mixed solution (internal temperature: 37°C).

The mixed solution was then stirred under cooling to gradually crystallize the monomaleic acid salt. 200 mL of t-butyl methyl ether was added dropwise to the mixed solution, and the mixture was stirred under heating and further stirred at an internal temperature of 40 to 45°C for 30 minutes. Subsequently, the mixed solution was gradually cooled and stirred at an internal temperature of 15°C or lower (to an internal temperature of 7°C) for 30 minutes. The precipitated crystal was collected by filtration, and washed with 50 mL of t-butyl methyl ether. A wet crystal was dried with blowing air at 40 to 50°C to obtain 8.36 g (yield: 84%) of white flocculent crystal of
2- [2-((S)-3-dimethylaminopyrrolidin-1-yl)pyridin-3-yl]-5-ethyl-7-methoxy-4H-benz [d][1,3]oxazin-4-one monomaleate.
Melting point (hot plate method): 153 to 163°C (melting)

### [Example 5]

A solution of maleic acid (50.4 mg) in water (1 mL) was mixed in a solution of 2-[2-((S)-3-dimethylaminopyrrolidin-1-yl)pyridin-3-yl]-5-ethyl-7-methoxy-4H-benz [d][1,3]oxazin-4-one (171 mg) in acetonitrile (2 mL) (hereinafter, the obtained solution was referred to as mixed solution), and the mixed solution was concentrated under reduced pressure.
Acetonitrile (5 mL) was added to an obtained concentrate of the mixed solution, and the mixture was concentrated under reduced pressure again. This concentration operation was repeated twice to get a residue as a solid.
The obtained solid was suspended in acetone (2 mL), a small amount of water was added at an external temperature of 45°C, and the solid was dissolved. Ethyl acetate (6 mL) was added to the solution, and the solution was cooled to room temperature. The precipitated crystal was collected by filtration, and the collected crystal was washed with ethyl acetate. The obtained crystal was dried with blowing air at 50°C to obtain 66.1 mg (yield: 30%) of 2- [2-((S)-3-dimethylaminopyrrolidin-1-yl)pyridin-3-yl]-5-ethyl-7-methoxy-4H-benz [d][1,3]oxazin-4-one monomaleate as white powder.
Melting point (hot plate method): 161 to 163°C (melting)

### [Comparative Example]

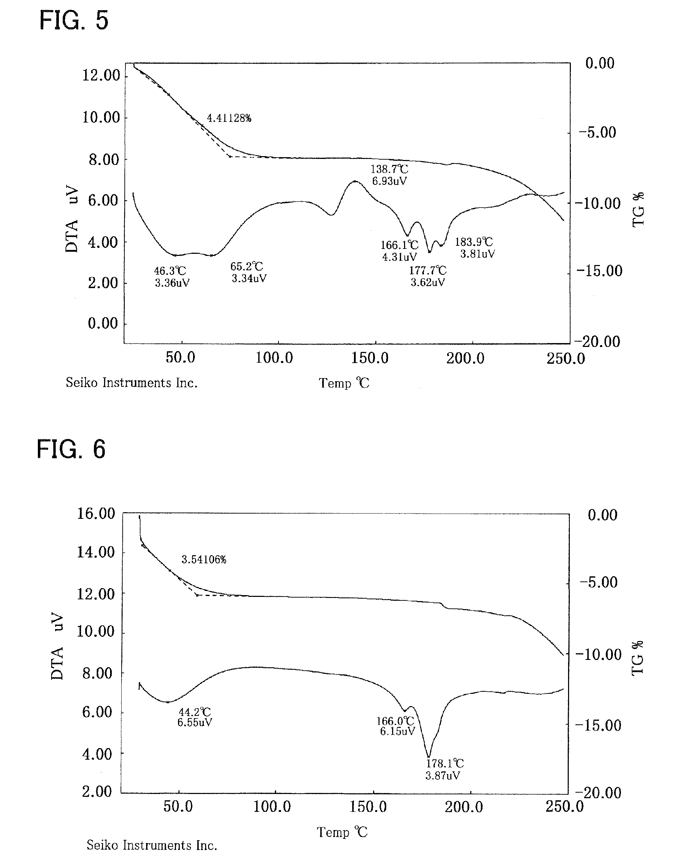

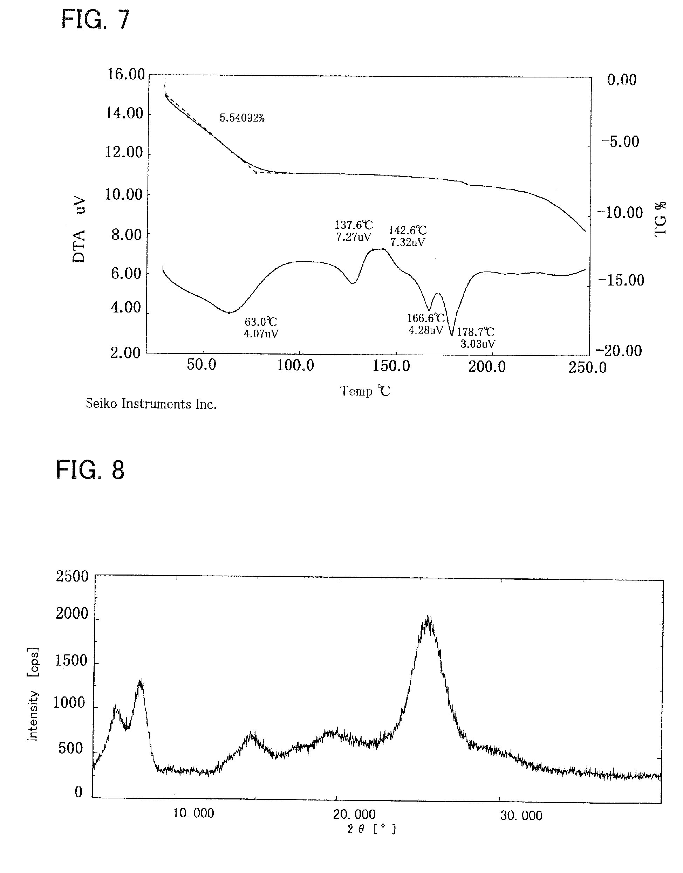

Crystal of hydrochloride monohydrate
Three lots of 2-[2-((S)-3-dimethylaminopyrrolidin-1-yl)pyridin-3-yl]-5-ethyl-7-methoxy-4H-benz [d][1,3]oxazin-4-one hydrochloride monohydrate were synthesized in accordance with Patent Literature 1.
Thermal analysis TG-DTA of each lot is shown (in Figs. 5, 6, and 7).
As seen from the results of performed powder x-ray diffractrion (Fig. 8), hydrochloride monohydrate in Comparative Example has low crystallinity and high amorphous content.

### [Test Example 1]

As the monomaleic acid salt, the sample in Example 5 was used in Stage 1 of Table 2, and the sample in Example 1 was used in Stage 2. The other samples in respective Stages of Table 2 were prepared in accordance with Patent Literature 1. The solubility and the stability as a drug substance of the respective samples were examined in Stage 1, and the crystallinity, phenomenon of crystal polymorphism, and presence or absence of adhesion water were examined in Stage 2.

Evaluation standards are as follows:
Solubility: When the ratio of dissolved sample to water is 10 mg/mL or more, the solubility of the sample is represented as circle (O), and when the ratio is 10 mg/mL or less, the solubility is represented as cross mark (×).
Stability as a drug substance: The sample after 2-week storage at 25°C/75%RT (closed system) was assayed by high-performance liquid chromatography (HPLC) under conditions described below. When the increase in area percentage (%) of a mainly degradation product is less than +0.5%, the stability of drug substances is represented as circle (O), and when the increase is +0.5% or more, the stability of drug substances is represented as cross mark (×). In Test Example 2, the mainly degradation product was assayed by HPLC under the same conditions.
Crystallinity: In powder x-ray diffraction, when a peak intensity is 5000 cps or more, the crystallinity is represented as "High," and when it is 5000 cps or less, the crystallinity is represented as "Low."
Development of crystal polymorphism: For crystals of the same salt, when the crystal structure shown by the thermal analysis TG-DTA is different from that shown by the powder x-ray diffraction, the development of crystal polymorphism is represented as "Presence". On the other hand, when the thermal analysis TG-DTA and the powder x-ray diffraction show the same crystal structure, the development of crystal polymorphism is represented as "None."
Adhesion water: When decrease (%) in the chart obtained from the thermal analysis TG-DTA is seen at about 50°C, the adhesion water is represented as "Presence," and when the decrease (%) is not seen, the adhesion water is represented as "None."

Conditions of HPLC in test of stability as a drug substance

### <Measurement Conditions>

column: Atlantis dC18, 2.1 mm φ × 15 cm, 3 µm
Measurement wavelength: 254 nm
column temperature: 40°C
Flow rate: 0.2 mL/min
Mobile phase A: 0.05% formic acid aqueous solution
Mobile phase B: 0.05% formic acid-acetonitrile solution
Transfer of mobile phases: The mixed ratio of mobile phase A and mobile phase B is changed as shown in Table 1 to control the concentration gradient.

**[Table 1]**

| TIME (MIN) | MOBILE PHASE A (%) | MOBILE PHASE B (%) |
|---|---|---|
| 0 ~ 23 | 80 → 60 | 20 → 40 |
| 23 ~ 25 | 60 → 30 | 40 → 70 |
| 25 ~ 28 | 30 | 70 |

**[Table 2]**

| EACH SALT | Stage 1 | | Stage 2 | | |
|---|---|---|---|---|---|
| | SOLUBILITY | STABILITY AS DRUG SUBSTANCE | CRYSTALLINITY | PHENOMENON OF CRYSTAL POLYMORPHISM | ADHESION WATER |
| FREE FORM | × | ○ | LOW | AMORPHOUS | - |
| MONOHYDROCHLORIDE | ○ | ○ | LOW | PRESENCE | PRESENCE |
| DIHYDROCHLORIDE | ○ | × | - | - | RESENCE |
| 1/2D-TARTARIC ACID | ○ | × | - | - | PRESENCE |
| 3/4L-TARTARIC ACID | × | ○ | - | - | PRESENCE |
| MONOMALEIC ACID | ○ | ○ | HIGH | PRESENCE | NONE |

| | | | | | |
|---|---|---|---|---|---|
| - unmeasured | | | | | |

The free base (free form) of 2-[2-((S)-3-dimethylaminopyrrolidin-1-yl)pyridin-3-yl]-5-ethyl-7-methoxy-4H-benz [d][1,3]oxazin-4-one was a yellow amorphous solid, and insoluble in water (2 mg/mL or less).
The 2-[2-((S)-3-dimethylaminopyrrolidin-1-yl)pyridin-3-yl]-5-ethyl-7-methoxy-4H-benz [d][1,3]oxazin-4-one hydrochloride monohydrate showed favorable solubility in water of 10 mg/mL or more, and the stability in 25°C/75%RH (closed system) was favorable since occurrence of degradation products after two weeks was less than 0.5%. However, it was confirmed from the results of performed powder x-ray diffraction that 2-[2-((S)-3-dimethylaminopyrrolidin-1-yl)pyridin-3-yl]-5-ethyl-7-methoxy-4H-benz [d][1,3]oxazin-4-one hydrochloride monohydrate had low crystallinity and high amorphous content (Fig. 8).
Further, from the results of performed thermal analysis TG-DTA, the decrease (%) in the amount at about 50°C was seen, and therefore it was understood that adhesion water was present.
Although the 1/2D-tartaric acid salt showed favorable solubility in water of 10 mg/mL or more, the stability in 25°C/75%RH (closed system) was poor, and therefore 0.5% or more of degradation products were generated after 2 weeks.
For a 3/4L-tartaric acid salt, the solubility in water was 2 to 5 mg/mL and therefore was insufficient.
Although the dihydrochloride showed favorable solubility in water of 10 mg/mL or more, the stability in 25°C/75%RH (closed system) was poor, and therefore 0.5% or more of degradation products were generated after 2 weeks.
On the other hand, the monomaleic acid salt of the present invention showed favorable solubility in water of 10 mg/mL or more. The stability in 25°C/75%RH (closed system) was good since occurrence of degradation products after 2 weeks was less than 0.5%. Further, as seen from the results of measured powder x-ray diffraction, it was confirmed that the monomaleic acid salt had high crystallinity (Fig. 2). Further, from the results of performed thermal analysis TG-DTA, the decrease (%) at about 50°C was not seen, and therefore it was understood that adhesion water was not present.

### [Test Example 2]

Each salt of 2-[2-((S)-3-dimethylaminopyrrolidin-1-yl)pyridin-3-yl]-5-ethyl-7-methoxy-4H-benz [d][1,3]oxazin-4-one was stored for 4 weeks under 4 conditions including 25°C/60%RH (closed system, shading), 40°C/75%RH (open system, shading), 40°C/75%RH (closed system, shading), and at room temperature under fluorescent lighting.
As samples, the same samples as in Test Example 1 (Stage 1) were used. The results of stability test are shown in Tables 3 and 4. Tables 3 and 4 show area percentage (%) of an amount of mainly degradation product which was measured by high-performance liquid chromatography (HPLC) at the respective measurement points.

**[Table 3]**

| STABILITY AS DRUG SUBSTANCE | | | | | | | |
|---|---|---|---|---|---|---|---|
| EACH SALT | start | 25°C/60%RH CLOSED SYSTEM SHADING | | | 40°C/75%RH OPEN SYSTEM SHADING | | |
| | | 1 WEEK | 2 WEEKS | 4 WEEKS | 1 WEEK | 2 WEEKS | 4 WEEKS |
| FREE FORM | 0.10% | - | .10% | 0.30% | - | 1.50% | 3.00% |
| MONOHYDROCHLORIDE | 0.10% | 0.20% | 0.20% | 0.20% | 0.20% | 0.30% | 0.50% |
| DIHYDROCHLORIDE | 6.40% | 7.90% | 15.40% | 12.20% | 8.20% | 19.40% | 24.00% |
| 1/2D-TARTARIC ACID | 1.10% | - | 2.40% | 3.40% | - | - | - |
| 3/4L-TARTARIC ACID | 0.00% | - | 0.10% | 0.10% | - | - | - |
| MONOMALEIC ACID | 0.00% | 0.00% | 0.00% | 0.00% | 0.00% | 0.00% | 0.10% |

**[Table 4]**

| STABILITY AS DRUG SUBSTANCE | | | | | | | |
|---|---|---|---|---|---|---|---|
| EACH SALT | start | 40°C/75%RH CLOSED SYSTEM SHADING | | | ROOM TEMPERATURE FLUORESCENT LIGHTING | | |
| | | 1 WEEK | 2 WEEKS | 4 WEEKS | 1 WEEK | 2 WEEKS | 4 WEEKS |
| FREE FORM | 0.10% | - | 1.50% | 3.20% | - | 0.10% | 0.30% |
| MONOHYDROCHLORIDE | 0.10% | 0.20% | 0.30% | 0.50% | 0.20% | 0.30% | 0.40% |
| DIHYDROCHLORIDE | 6.40% | 13.90% | 27.70% | 15.50% | 8.00% | 17.90% | 20.60% |
| 1/2D-TARTARIC ACID | 1.10% | - | 7.60% | 16.10% | - | 2.60% | 4.10% |
| 3/4L-TARTARIC ACID | 0.00% | - | 0.10% | 0.10% | - | 0.10% | 0.10% |
| MONOMALEIC ACID | 0.00% | 0.00% | 0.00% | 0.10% | 0.00% | 0.00% | 0.00% |

From Tables 3 and 4, for the free form, dihydrochloride, and 1/2D-tartaric acid salt, the amount of mainly degradation product after 4-week storage exceeded 1%, and therefore it is understood that they are unstable. On the other hand, the monohydrochloride, 3/4L-tartaric acid salt, and monomaleic acid salt had an amount of mainly degradation product of 1% or less, and therefore were excellent in stability.
Further, even in the case of 4-week storage at room temperature under fluorescent lighting, the monohydrochloride and 3/4L-tartaric acid salt had an amount of mainly degradation product of 1% or less, and therefore were excellent in stability, but the total amounts of degradation products were 8.2% and 1.4%, respectively. On the other hand, the monomaleic acid salt had a total amount of degradation product of 0.2%, and therefore was further excellent in stability.

### Industrial Applicability

A compound excellent in storage stability (decomposition is less), solubility, crystallinity, and easy of handling (mass is not increased or decreased by moisture absorption or desorption) can be obtained by the present invention. The present invention can provide a drug substance stable in quality.

## Claims

1. Maleic acid salt of 2-[2-((S)-3-dimethylaminopyrrolidin-1-yl)pyridin-3-yl)-5-ethyl-7-methoxy-4H-benz [d][1,3]oxazin-4-one represented by the formula (1),

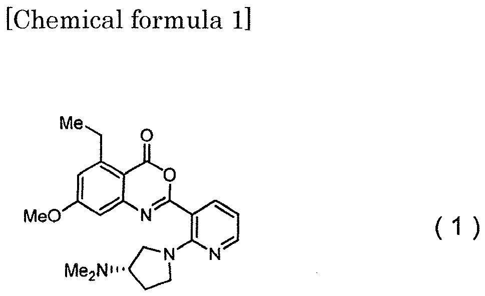

2. A crystal of 2-[2-((S)-3-dimethylaminopyrrolidin-1-yl)pyridin-3-yl]-5-ethyl-7-methoxy-4H-benz [d][1,3]oxazin-4-one maleate.

3. A crystal of 2-[2-((S)-3-dimethylaminopyrrolidin-1-yl)pyridin-3-yl]-5-ethyl-7-methoxy-4H-benz [d][1,3]oxazin-4-one monomaleate.

4. The crystal of 2-[2-((S)-3-dimethylaminopyrrolidin-1-yl)pyridin-3-yl]-5-ethyl-7-methoxy-4H-benz [d][1,3]oxazin-4-one monomaleate according to claim 3, having a characteristic peak at a diffraction angle (2θ ± 0.5°) of 10.8° in powder x-ray diffraction.

5. The crystal of 2-[2-((S)-3-dimethylaminopyrrolidin-1-yl)pyridin-3-yl]-5-ethyl-7-methoxy-4H-benz [d][1,3]oxazin-4-one monomaleate according to claim 3, having a characteristic peak at a diffraction angle (2θ ± 0.5°) of 14.8° in powder x-ray diffraction.

6. The crystal of 2-[2-((S)-3-dimethylaminopyrrolidin-1-yl)pyridin-3-yl]-5-ethyl-7-methoxy-4H-benz [d][1,3]oxazin-4-one monomaleate according to claim 4, wherein melting point thereof measured by thermal analysis TG-DTA is about 153°C.

7. The crystal of 2-[2-((S)-3-dimethylaminopyrrolidin-1-yl)pyridin-3-yl]-5-ethyl-7-methoxy-4H-benz [d][1,3]oxazin-4-one monomaleate according to claim 5, wherein melting point thereof measured by the thermal analysis TG-DTA is about 167°C.

8. A mixture of a crystal of 2-[2-((S)-3-dimethylaminopyrrolidin-1-yl)pyridin-3-yl]-5-ethyl-7-methoxy-4H-benz [d] [1,3]oxazin-4-one monomaleate having a characteristic peak at a diffraction angle (2θ ± 0.5°) of 10.8° in powder x-ray diffraction, and
a crystal of 2-[2-((S)-3-dimethylaminopyrrolidin-1-yl)pyridin-3-yl]-5-ethyl-7-methoxy-4H-benz [d][1,3]oxazin-4-one monomaleate having a characteristic peak at a diffraction angle (2θ ± 0.5°) of 14.8° in powder x-ray diffraction.

9. A method for producing the crystal of 2-[2-((S)-3-dimethylaminopyrrolidin-1-yl)pyridin-3-yl]-5-ethyl-7-methoxy-4H-benz [d][1,3]oxazin-4-one monomaleate according to claim 4, comprising the following steps (i) and (ii):
(i) a step of dissolving a 2-[2-((S)-3-dimethylaminopyrrolidin-1-yl)pyridin-3-yl]-5-ethyl-7-methoxy-4H-benz [d][1,3]oxazin-4-one free base in acetone to produce a solution; and
(ii) a step of adding a solution of maleic acid in acetone to the solution obtained in the step (i).

10. A method for producing the crystal of 2-[2-((S)-3-dimethylaminopyrrolidin-1-yl)pyridin-3-yl]-5-ethyl-7-methoxy-4H-benz [d][1,3]oxazin-4-one monomaleate according to claim 4, comprising
using an aprotic organic solvent as a solvent in which a 2-[2-((S)-3-dimethylaminopyrrolidin-1-yl)pyridin-3-yl]-5-ethyl-7-methoxy-4H-benz [d][1,3]oxazin-4-one free base and maleic acid are dissolved, and
using one or more diluting solvents selected from the group consisting of ethyl acetate, t-butyl methyl ether, tetrahydrofuran, diethyl ether, and diisopropyl ether as a solvent to be added for precipitation of crystals.

11. A method for recrystallizing 2-[2-((S)-3-dimethylaminopyrrolidin-1-yl)pyridin-3-yl]-5-ethyl-7-methoxy-4H-benz [d][1,3]oxazin-4-one monomaleate, comprising the following steps (i) to (iii):
(i) a step of dissolving a 2-[2-((S)-3-dimethylaminopyrrolidin-1-yl)pyridin-3-yl]-5-ethyl-7-methoxy-4H-benz [d][1,3]oxazin-4-one monomaleate in a mixed solvent of acetone and water to produce a solution;
(ii) a step of adding t-butyl methyl ether or diisopropyl ether to the solution obtained in the step (i); and
(iii) a step of isolating crystalline solid crystallized by the step (ii).

12. A pharmaceutical comprising the crystal of 2- [2-((S)-3-dimethylaminopyrrolidin-1-yl)pyridin-3-yl]-5-ethyl-7-methoxy-4H-benz [d][1,3]oxazin-4-one monomaleate according to claim 3 as a drug substance.
